# EUROPÄISCHE PATENTSCHRIFT

(11) **EP 2 295 051 B1**
(45) Veröffentlichungstag und Bekanntmachung des Hinweises auf die Patenterteilung: **27.11.2013**
(21) Anmeldenummer: 10171229.7
(22) Anmeldetag: 29.07.2010
(51) Int. Cl.: A61K 31/185, A61K 33/10, A61P 17/02

(54) **Wundheilzusammensetzung**
Wound healing compound
Composition pour cicatrisation de plaies

(30) Priorität: 29.07.2009 DE 102009035109
(43) Veröffentlichungstag der Anmeldung: 16.03.2011
(73) Patentinhaber: Kraus, Ferki, 84034 Landshut (DE)
(72) Erfinder: Kraus, Ferki, 84034 Landshut (DE)
(74) Vertreter: Bittner, Bernhard

(56) Entgegenhaltungen:
- US-A1- 2005 112 208
- DATABASE WPI Week 200010 Thomson Scientific, London, GB; AN 2000-106959 XP002602429 & CN 1 232 700 A (GAO Y) 27. Oktober 1999 (1999-10-27)
- DATABASE WPI Week 200430 Thomson Scientific, London, GB; AN 2004-326118 XP002602430 & RU 2 223 751 C1 (KOZLOVICH A V) 20. Februar 2004 (2004-02-20)
- CHICK L R ET AL: "Calcium carbonate gel therapy for hydrofluoric acid burns of the hand" PLASTIC AND RECONSTRUCTIVE SURGERY, WILLIAMS AND WILKINS CO., BALTIMORE, MD, US LNKD- DOI:10.1097/00006534-199011000-00016, Bd. 86, Nr. 5, 1. November 1990 (1990-11-01), Seiten 935-940, XP008101180 ISSN: 0032-1052
- DATABASE WPI Week 200910 Thomson Scientific, London, GB; AN 2009-B38539 XP002602431 & RU 2 337 702 C2 (GAYSANOVA L I) 10. November 2008 (2008-11-10)
- ANONYMOUS 27 Oktober 1999, INTERNET, XP055030044

## Beschreibung

Die vorliegende Erfindung bezieht sich auf eine Wundheilzusammensetzung zur Behandlung von Brandwunden. Derartige Wundheilzusammensetzungen sind in Form von Salben, Pasten oder dergleichen bekannt. Dabei können diese Wundheilzusammensetzungen unterschiedliche Substanzen aufweisen.

Der vorliegenden Erfindung liegt die Aufgabe zugrunde, eine Wundheilzusammensetzung zur Verfügung zu stellen, welche einerseits hohe Wirksamkeit gegen Brandwunden entfaltet und andererseits aus hautverträglichen Substanzen zusammengesetzt ist. Weiterhin liegt der Erfindung die Aufgabe zugrunde, ein Verfahren für die Herstellung einer derartigen Wundheilzusammensetzung zur Verfügung zu stellen.

Aus der CN 1232700 ist eine Zubereitung bekannt, welche Vaseline, Sesamöl, Calciumcarbonat-Creme und Borneol enthält.

Eine erfindungsgemäße Wundheilzusammensetzung zur Behandlung von Brandwunden weist sowohl von Flüssigkeit durchsetzten Kalk als auch ein Öl auf, wobei der von Flüssigkeit durchsetzte Kalk sowie das Öl miteinander vermischt sind.

Die auf diese Weise hergestellte Substanz lässt sich auf Brandwunden auftragen und bewirkt innerhalb kurzer Zeit einen Heilungserfolg. Unter Brandwunden werden im Rahmen dieser Anmeldung nicht nur offene Wunden im engeren Sinne sondern vielmehr auch jegliche Arten von Verbrennungen, insbesondere der menschlichen Haut verstanden.

Vorteilhaft liegt der Volumenanteil an Öl in der Zusammensetzung zwischen 20 % und 80 %, vorteilhaft zwischen 30 % und 70 % und besonders bevorzugt zwischen 40 % und 60 %.

Bevorzugt liegt auch der Volumenanteil an von Flüssigkeit durchsetztem Kalk in der Zusammensetzung zwischen 20 % und 80 %, bevorzugt zwischen 30 % und 70 % und besonders bevorzugt zwischen 40 % und 60 %. Besonders bevorzugt liegt das Verhältnis zwischen dem Öl und dem von Flüssigkeit durchsetzten Kalk (CaCO₃) zwischen 4 : 6 und 6 : 4. Bei dieser Zusammensetzung wird einerseits eine besonders günstige Mischbarkeit erreicht und andererseits die Auftragbarkeit der Salbe erleichtert, bzw. eine für die Auftragung günstige Viskosität der entstehenden Zusammensetzung erreicht.

Besonders vorteilhaft ist der Kalk von Wasser durchsetzt oder mit Wasser gemischt. Wasser eignet sich in besonders vorteilhafter Weise zum Durchsetzen des Kalks, da es einerseits unbedenklich für die Haut ist und andererseits auch sehr leicht beschafft werden kann. Daneben ist Kalk in Wasser nur schwer lösbar. Bevorzugt wird daher als Flüssigkeit eine solche Flüssigkeit verwendet, in der sich Kalk nur unwesentlich löst.

Weiterhin ist vorteilhafter Weise das Öl aus einer Gruppe von Ölen ausgewählt, welches Sonnenblumenöl, Rapsöl, Olivenöl und dergleichen enthält. Allgemein handelt es sich bevorzugt bei dem Öl, welches aus Naturprodukten gewonnen wird.

Besonders vorteilhaft handelt es sich bei dem Öl um ein Olivenöl.

Weiterhin weist vorteilhaft das Öl einen Ölsäureanteil auf, der zwischen 50 % und 90 %, bevorzugt zwischen 60 % und 80 % liegt. Olivenöl besteht, wie alle Pflanzenöle, aus an Glycerin gebundenen Fettsäuren. Im Olivenöl sind durchschnittlich 72 % Ölsäure, 7, 9 % Linolsäure, 11 % Palmitinsäure, 2,2 % Stearinsäure und 5 % Palmitoleinsäure enthalten.

Die vorliegende Erfindung ist weiterhin auf ein Verfahren zur Herstellung einer Wundheilzusammensetzung zur Behandlung von Brandwunden und insbesondere der oben beschriebenen Wundheilzusammensetzung gerichtet. Dabei wird in einem ersten Verfahrensschritt Kalk zur Verfügung gestellt und in einem weiteren Verfahrensschritt der Kalk mit einer Flüssigkeit in einem Behältnis gemischt. Schließlich wird der so entstandenen Mischung ein Öl zugemischt. Dabei ist es wiederum möglich, dass das Öl der Mischung zugegeben wird, es wäre jedoch auch möglich, dass das Öl zunächst in ein Behältnis eingefüllt und anschließend die Mischung zugegeben wird.

Vorteilhaft wird bewirkt, dass der Kalk von der genannten Flüssigkeit durchsetzt wird, wobei es sich bei dieser Flüssigkeit, wie oben erwähnt, besonders bevorzugt um Wasser handelt.

Dabei wäre es auch möglich, sterilisiertes Wasser zum Durchsetzen des Kalks zu verwenden. Bei einem möglichen Verfahren wird zunächst der Kalk in ein Behältnis gefüllt und anschließend die Flüssigkeit bzw. Wasser in dieses Behältnis eingefüllt. Es wäre jedoch auch möglich, dass der Kalk der Flüssigkeit zugeführt und bevorzugt mit dieser vermischt wird. Auch könnten wechselweise Kalk und Flüssigkeit zugeführt werden.

Bei einem weiteren vorteilhaften Verfahren wird nach dem Mischen von Kalk mit der Flüssigkeit in dem Behältnis ein vorgegebener Anteil der Flüssigkeit wieder abgenommen. So wäre es möglich, dass der Kalk mit einer größeren Menge an Flüssigkeit durchsetzt wird als dies an sich nötig wäre und anschließend ein Anteil der Flüssigkeit wieder abgenommen wird. Vorteilhaft wird während der Zugabe von Flüssigkeit zu dem Kalk die so entstehende Substanz - bevorzugt permanent - gemischt.

Bei einem weiteren vorteilhaften Verfahren wird nach dem Mischen von Kalk mit der Flüssigkeit in dem Behältnis die genannte Mischung einer Ruhephase unterworfen. In dieser Ruhephase kann sich der Kalk absetzen, bleibt jedoch nach wie vor von der Flüssigkeit durchsetzt. Auf diese Weise ist es besonders bevorzugt möglich, dass nach der Ruhephase der Anteil an klarer Flüssigkeit aus dem Behältnis abgenommen wird. Dabei ist es möglich, dass diese klare Flüssigkeit abgeschöpft wird, sie könnte jedoch auch abgeschüttet oder mit einem Schlauch abgeführt werden.

Vorteilhaft ist der Volumenanteil an Flüssigkeit größer als der Volumenanteil an Kalk. Auf diese Weise wird eine besonders vorteilhafte Durchsetzung des Kalks mit Flüssigkeit erreicht. Vorteilhaft stehen der Volumenanteil an Flüssigkeit und der Volumenanteil an Kalk in einem Verhältnis von mindestens 60 zu 40, bevorzugt von ca. 70 zu 30.

Bei einem weiteren vorteilhaften Verfahren beträgt die Ruhephase wenigstens 8 Stunden, bevorzugt wenigstens 14 Stunden und besonders bevorzugt wenigstens 24 Stunden. Diese vergleichsweise lange Zeit dient besonders bevorzugt dazu, um die Restflüssigkeit in möglichst klarer Konsistenz abnehmen zu können. Mit anderen Worten kann sich während dieser Ruhezeit der ungelöste oder nahezu ungelöste Kalk am Boden des Gefäßes absetzen. Es wird daher bevorzugt eine Flüssigkeit verwendet, deren spezifisches Gewicht geringer ist als dasjenige von Kalk.

Bei einem weiteren vorteilhaften Verfahren wird der Kalk als Kalkstein oder in Pulverform zur Verfügung gestellt. Insbesondere die Pulverform eignet sich in besonders günstiger Weise zum Herstellen der Wundheilzusammensetzung.

Bei einem weiteren bevorzugten Verfahren wird der von Flüssigkeit durchsetzte Kalk mit dem Öl vermischt. Diese Vermischung erfolgt dabei vorteilhaft nach dem Abnehmen des klaren Wassers. Auf diese Weise kann die Viskosität der Zusammensetzung erhöht werden, um auf diese Weise ein Auftragen auf Brandwunden zu erleichtern.

Die vorliegende Erfindung ist weiterhin auf die Verwendung einer Wundheilzusammensetzung nach wenigstens einem der vorangegangenen Ansprüche zum Behandeln von Brandwunden gerichtet. Vorzugsweise wird die Wundheilzusammensetzung zum Behandeln von Verbrennungen ersten oder zweiten Grades verwendet.

## Patentansprüche

1. Wundheilzusammensetzung, welche zur Verwendung bei der Behandlung von Brandwunden geeignet ist, wobei diese Zusammensetzung sowohl mit Wasser gemischten Kalk als auch ein Öl enthält und der mit Wasser gemischte Kalk sowie das Öl miteinander vermischt sind
**dadurch gekennzeichnet, dass**
der Kalk in Wasser gemischt und der Volumenanteil an mit Wasser gemischtem Kalk in der Zusammensetzung zwischen 40% und 60% liegt und der Volumenanteil an Öl in der Zusammensetzung zwischen 40% und 60% liegt, derart, dass ein Verhältnis zwischen dem Öl und dem von Wasser durchsetzten Kalk zwischen 4:6 und 6:4 liegt, wobei der Volumenanteil an Wasser und der Volumenanteil an Kalk in einem Verhältnis von mindestens 60 zu 40 stehen.

2. Wundheilzusammensetzung nach Anspruch 1
**dadurch gekennzeichnet, dass**
das Öl aus einer Gruppe von Ölen ausgewählt ist, welche Sonnenblumenöl, Rapsöl, Olivenöl und dergleichen ist.

3. Wundheilzusammensetzung nach Anspruch 1
**dadurch gekennzeichnet, dass**
das Öl einen Ölsäurenanteil aufweist, der zwischen 50% und 90%, bevorzugt zwischen 60% und 80% liegt.

4. Verfahren zur Herstellung einer Wundheilzusammensetzung, welche zur Verwendung der Behandlung von Brandwunden geeignet ist und insbesondere einer Wundheilzusammensetzung nach wenigstens einem der vorangegangenen Ansprüche mit den Schritten:
- Zur Verfügung - Stellung von Kalk;
- Mischen von Kalk und Wasser in einem Behältnis;
- Zumischen eines Öls, sodass der Volumenanteil an mit Wasser gemischtem Kalk in der Zusammensetzung zwischen 40% und 60% liegt und der Volumenanteil an Öl in der Zusammensetzung zwischen 40% und 60% liegt, derart, dass ein Verhältnis zwischen dem Öl und dem von Wasser durchsetzten Kalk zwischen 4:6 und 6:4 liegt, wobei der Volumenanteil an Wasser und der Volumenanteil an Kalk in einem Verhältnis von mindestens 60 zu 40 stehen.

5. Verfahren nach Anspruch 4,
**dadurch gekennzeichnet, dass**
nach dem Mischen von Kalk mit dem Wasser in dem Behältnis ein vorgegebener Anteil des Wassers wieder abgenommen wird.

6. Verfahren nach wenigstens einem der vorangegangenen Ansprüche 4 - 5,
**dadurch gekennzeichnet, dass**
nach dem Mischen von Kalk mit dem Wasser in dem Behältnis die Mischung einer Ruhephase unterworfen wird.

7. Verfahren nach Anspruch 6,
**dadurch gekennzeichnet, dass**
nach der Ruhephase der Anteil an klarem Wasser abgenommen wird.

8. Verfahren nach Anspruch 6,
**dadurch gekennzeichnet, dass**
der Volumenanteil an Wasser größer ist als der Volumenanteil an Kalk

9. Verfahren nach wenigstens einem der vorangegangenen Ansprüche 6 - 8
**dadurch gekennzeichnet, dass**
die Ruhephase wenigstens 8 Stunden, bevorzugt wenigstens 14 Stunden, besonders bevorzugt wenigstens 24 Stunden beträgt.

10. Verfahren nach wenigstens einem der Ansprüche 4 - 9,
**dadurch gekennzeichnet, dass**
der Kalk als Kalkstein oder in Pulverform zur Verfügung gestellt wird.

11. Verfahren nach wenigstens einem der Ansprüche 4 - 9,
**dadurch gekennzeichnet, dass**
der in Wasser getränkte Kalk mit dem Öl vermischt wird.

12. Wundheilzusammensetzung nach Anspruch 1
zur Verwendung bei der Behandlung von Brandwunden.

## Claims

1. A wound healing composition which is suitable for use in the treatment of burn wounds, wherein this composition contains both lime mixed with water and an oil and the lime mixed with water as well as the oil are mixed together, **characterized in that** the lime is mixed in water and the volume ratio of lime mixed with water in the composition is between 40 % and 60 % and the volume ratio of oil in the composition is between 40 % and 60 %, in such a way that a ratio between the oil and the lime permeated by water is between 4 : 6 and 6 : 4, wherein the volume ratio of water and the volume ratio of lime is in a ratio of at least 60 : 40.

2. A wound healing composition according to claim 1, **characterized in that** the oil is selected from a group of oils which is sunflower oil, rapeseed oil, olive oil and the like.

3. A wound healing composition according to at least one of the preceding claims, **characterized in that** the oil has a portion of oleic acid which is between 50 % and 90 %, preferably between 60 % and 80 %.

4. A method of preparing a wound healing composition which is suitable for use in the treatment of burn wounds and in particular a wound healing composition according to at least one of the preceding claims, with the steps:
- the making available of lime;
- the mixing of lime and water in a container;
- the admixing of an oil, so that the volume ratio of lime mixed with water in the composition is between 40 % and 60 % and the volume ratio of oil in the composition is between 40 % and 60 %, in such a way that a ratio between the oil and the lime permeated by water is between 4 : 6 and 6 : 4, wherein the volume ratio of water and the volume ratio of lime is in a ratio of at least 60 : 40.

5. A method according to claim 4, **characterized in that** after the mixing of lime with the water in the container a pre-set portion of the water is removed again.

6. A method according to at least one of the preceding claims 4 to 5, **characterized in that** after the mixing of lime with the water in the container the mixture undergoes a rest phase.

7. A method according to claim 6, **characterized in that** after the rest phase the portion of clear water is removed.

8. A method according to claim 6, **characterized in that** the volume ratio of water is greater than the volume ratio of lime.

9. A method according to at least one of the preceding claims 6 to 8, **characterized in that** the rest phase amounts to at least 8 hours, preferably at least 14 hours, and in a particularly preferred manner at least 24 hours.

10. A method according to at least one of claims 4 to 9, **characterized in that** the lime is made available in the form of limestone or in a powdered form.

11. A method according to at least one of claims 4 to 9, **characterized in that** the lime impregnated in water is mixed with the oil.

12. A wound healing composition according to claim 1 for use in the treatment of burn wounds.

## Revendications

1. Composition cicatrisante, qui est appropriée à l'utilisation lors du traitement des brûlures, cette composition comprenant aussi bien de la chaux mélangée à de l'eau qu'une huile, la chaux mélangée à l'eau et l'huile étant mélangées entre elles,
**caractérisée en ce que**
la chaux mélangée dans de l'eau et la proportion volumique de chaux mélangée à l'eau dans la composition se situe entre 40 % et 60 % et la proportion volumique d'huile dans la composition se situe entre 40 % et 60 % de sorte qu'un rapport entre l'huile et la chaux mélangée à l'eau se situe entre 4/6 et 6/4, la proportion volumique d'eau et la proportion volumique de chaux se situant dans un rapport d'au moins 60 : 40 %.

2. Composition cicatrisante selon la revendication 1, **caractérisée en ce que** l'huile est choisie dans un groupe d'huiles qui est l'huile de tournesol, l'huile de colza, l'huile d'olive et similaire.

3. Composition cicatrisante selon au moins l'une des revendications précédentes, **caractérisée en ce que** l'huile présente une proportion d'acide oléique qui se situe entre 50 % et 90 %, de préférence entre 60 % et 80 %.

4. Procédé de préparation d'une composition cicatrisante, qui est appropriée à l'utilisation lors du traitement des brûlures et notamment d'une composition cicatrisante selon au moins l'une des revendications précédentes comprenant les étapes consistant à :
- mettre à disposition de la chaux ;
- mélanger de la chaux et de l'eau dans un récipient ;
- ajouter une huile, de sorte que la proportion volumique de chaux mélangée à l'eau dans la composition se situe entre 40 % et 60 % et la proportion volumique d'huile dans la composition se situe entre 40 % et 60 % de sorte qu'un rapport entre l'huile et la chaux mélangée à l'eau se situe entre 4/6 et 6/4, la proportion volumique d'eau et la proportion volumique de chaux se situant dans un rapport d'au moins 60 : 40 %.

5. Procédé selon la revendication 4, **caractérisé en ce que**, après le mélange de la chaux et de l'eau dans le récipient, une proportion prédéterminée d'eau est de nouveau retirée.

6. Procédé selon au moins l'une des revendications précédentes 4 à 5, **caractérisé en ce que**, après le mélange de la chaux et de l'eau dans le récipient, le mélange est soumis à une phase de repos.

7. Procédé selon la revendication 6, **caractérisé en ce que**, après la phase de repos, la proportion d'eau claire est retirée.

8. Procédé selon la revendication 6, **caractérisé en ce que** la proportion volumique d'eau est supérieure à la proportion volumique de chaux.

9. Procédé selon au moins l'une des revendications précédentes 6 à 8, **caractérisé en ce que** la phase de repos est au moins de 8 heures, de préférence au moins de 14 heures, particulièrement préférentiellement d'au moins 24 heures.

10. Procédé selon au moins l'une des revendications 4 à 9, **caractérisé en ce que** la chaux est mise à disposition sous forme de pierre ou de poudre.

11. Procédé selon au moins l'une des revendications 4 à 9, **caractérisé en ce que** la chaux imprégnée d'eau est mélangée à l'huile.

12. Composition cicatrisante selon la revendication 1 pour l'utilisation lors du traitement des brûlures.
